# EUROPÄISCHE PATENTSCHRIFT

(11) **EP 4 162 889 B1**
(45) Veröffentlichungstag und Bekanntmachung des Hinweises auf die Patenterteilung: **19.03.2025**
(21) Anmeldenummer: 22194730.2
(22) Anmeldetag: 09.09.2022
(51) Int. Cl.: A61B 17/80

(54) **IMPLANTATSYSTEM**
IMPLANT SYSTEM
SYSTÈME D'IMPLANT

(30) Priorität: 04.10.2021 DE 102021211157
(43) Veröffentlichungstag der Anmeldung: 12.04.2023
(73) Patentinhaber: Karl Leibinger Asset Management GmbH & Co. KG, 78570 Mühlheim an der Donau (DE)
(72) Erfinder: AKSU, Adem, 78054 VS-Schwenningen (DE); IRION, Veit, 78570 Muehlheim a. d. Donau (DE); REINAUER, Frank, 78576 Emmingen-Liptingen (DE)
(74) Vertreter: Isarpatent

(56) Entgegenhaltungen:
- EP-A1- 0 014 823
- US-A1- 2005 085 814
- US-A1- 2012 203 284
- US-A1- 2017 065 317
- US-A1- 2021 298 808

## Beschreibung

Die vorliegende Erfindung betrifft ein Implantatsystem zur Versorgung von Frakturen einer gekrümmten Knochenstruktur, insbesondere von gebrochenen Rippen im Thoraxbereich. Außerdem betrifft die Erfindung ein Verfahren zur Herstellung eines solchen Implantatsystems.

Die US 2021/0298808 A1 offenbart eine Sternum-Ersatzplatte mit einem zentralen Bereich und stabartigen Erweiterungen. Die Erweiterungen sind dicker als der zentrale Bereich, so dass der zentrale Bereich um eine Biegeachse verbiegbar ist. Die US 2005/085814 A1 betrifft eine orthopädische Vorrichtung zur Befestigung mehrerer Knochenteile aneinander, insbesondere Wirbelsäule. Die Vorrichtung umfasst eine stabartige Hauptstruktur, deren beide Enden mit Befestigungsstrukturen (z.B. Bohrungen, in die Bolzen eingeführt werden können) versehen sind. Die Enden sind mit einem drehbaren und ausziehbaren Verbindungsstück verbunden, das eine relative Drehung und Dehnung der Enden ermöglicht. Das Verbindungsstück ist mit einem biodegradierbaren Verstärkungsmaterial umhüllt, das der Bewegung der Enden Widerstand entgegensetzt und allmählich nachgibt, als das Verstärkungsmaterial resorbiert wird.

Die US 2012/203284 A1 beschreibt eine Befestigungsvorrichtung mit Arretierungsmitteln an beiden Enden und eine Verbindungsstruktur in der Mitte, die Akkordeon-artig dehnbar ist. Damit ist der Abstand der Enden zueinander regulierbar. Die US 2018/0360506 A1 offenbart eine Knochenfixierungsvorrichtung mit zwei starren Endstücken, welche über einen elastischen Verbindungsbereich aus Drähten verbunden sind.

Unter einem Thoraxtrauma versteht man eine Verletzung des knöchernen Brustkorbs sowie sämtlicher darin enthaltener Organe und Organsysteme. Thoraxtraumata entstehen überwiegend im Rahmen großer Gewalteinwirkungen mit Verletzungen mehrerer Körperregionen.

Während man isolierte Thoraxtraumen selten antrifft, liegt bei fast der Hälfte der Patienten, die ein Polytrauma erleiden, simultan ein Thoraxtrauma vor. Von einem Polytrauma spricht man, wenn gleichzeitig mehrere Körperregionen oder Organsysteme betroffen sind, wobei bereits eine der Verletzungen alleine oder die Kombination der Verletzungen lebensbedrohlich ist.

Der Thorax besteht aus der Brustwirbelsäule, dem Sternum und 12 paarig angelegte Rippen. Kranial wird der Thorax durch den oberen Brustkorbeingang begrenzt, der aus dem ersten Brustwirbelkörper, den beiden ersten Rippen und dem oberen Anteil des Sternums gebildet wird.

Er stellt die Verbindung zwischen den Bindegewebsräumen des Halses und des Brustkorbs dar. Die kaudale Begrenzung des Thorax, der untere Brustkorbeingang stellt die Grenze zwischen Brustraum und Bauchraum dar. Er wird durch den 12. Brustwirbelkörper, die 11. und 12. Rippe, die Knorpelanteile der 7. bis 10. Rippe sowie durch den Unterrand des Sternums dargestellt. Die Grenzstruktur zwischen Brust- und Bauchraum ist das Diaphragma das zwischen den genannten Skelettanteilen aufgespannt ist. Die Rippen sind sowohl mit der Wirbelsäule über sogenannte Kostovertebralgelenke, als auch mit dem Brustbein über sogenannte Sternokostalgelenke verbunden.

Im Zusammenspiel mit der zwischen den Rippen liegenden Brustkorbmuskulatur, die die Bereiche zwischen den Rippen, die sogenannte Interkostalräume, ausfüllt, nehmen die Gelenke eine zentrale Bedeutung in der Atemmechanik ein. Da der Brustkorb außerdem lebenswichtige Organe wie Herz, Lunge und wichtige Leitungsbahnen beherbergt, dient er somit auch als Schutzgerüst.

Während des Einatmens werden durch Dehnungen der Rippenköpfchen in den Kostovertebralgelenken die ventralen, sternalen Rippenenden angehoben. Parallel dazu tritt das Zwerchfell durch Kontraktion tiefer. Dies hat eine Volumenzunahme des Thorax zur Folge. Bei der Ausatmung senken sich die ventralen sternalen Rippenenden wieder ab und die Zwerchfellmuskulatur entspannt, somit der Thorax sein ursprüngliches Volumen wieder erreicht. Von allen Frakturen im Bereich des Thorax ist die Rippenfraktur die häufigste.

Derartige Frakturen wie solche der Rippen können beispielsweise durch Metallimplantate zur Versorgung und Knochenrekonstruktion eingesetzt werden. Hierbei kennt man etwa Kirschnerdrähte, die im Wege einer sogenannten K-Draht-Osteosynthese in der Lage sind, die betreffenden Frakturen zu fixieren. Diese können jedoch nach Einbringung in den Knochen wandern, insbesondere in Knochenhohlräumen und sind auch nicht versagensfrei. Weiter ist beim Einsatz von K-Drähten zur Ruhigstellung oft eine zusätzliche Gipsverbandfixierung notwendig, was die Versorgung von Thoraxtraumata erschwert.

Weiter kennt man zur Frakturversorgung Metallschienen, die jedoch nur eine ungenügende Schmerzausschaltung ermöglichen. Vollständig kann diese bei Rippenfrakturen auch mit Rippenklammern nicht erreicht werden, mit denen sich überdies eine kontrollierte Bewegung im Frakturspalt nicht erreichen lässt. Insgesamt ist auch hier eine primäre Knochenheilung jedenfalls stark erschwert.

Eine weitere Möglichkeit zur Fixierung der erwähnten Frakturen ist durch Plattenosteosynthese mittels Platten oder Schienen gegeben. In diesem Zusammenhang kennt man aus der DE 38 03 435 C1 eine Implantatschiene mit an die Kontur einer Rippe angepasster Seitenfläche. Nachteilig bei der Plattenosteosynthese kann sein, dass durch die Steifigkeit des Implantats die Atemmechanik des Rippenthorax beeinträchtigt wird. Insbesondere bei osteoporotischen Knochen können darüber hinaus Lockerungen schraubenartiger Befestigungen am Übergang zum nicht-steifen Rippenbereich auftreten.

Es ist daher die Aufgabe der vorliegenden Erfindung, ein Implantatsystem zur Verfügung zu stellen, welches eine Fraktur an einer Knochenstruktur sicher und versagensfrei versorgt und gleichzeitig bei guter Knochenheilung und Schmerzausschaltung eine kontrollierte Bewegung im Frakturspalt ermöglicht.

Die Aufgabe wird durch ein Implantatsystem mit den Merkmalen des Anspruchs 1 gelöst.

Mit dem erfindungsgemäßen Implantatsystem kann ein Implantatkörper auf eine Weise an der gekrümmten Knochenstruktur angeordnet werden, die bspw. durch Biegung die Krümmung der Knochenstruktur abbildet oder die Knochenstruktur durch Torsion des wenigstens einen Mittelstücks zumindest teilweise einfasst oder umschlingt, so dass einerseits die Fragmente der Knochenstruktur sicher gehalten sind und dabei andererseits eine kontrollierte Bewegung der Knochenstruktur im Ganzen ermöglicht wird, ohne den Heilungsprozess zu behindern, das Implantatsystem also ein kontrolliertes elastisches Bewegungsverhalten der betroffenen Knochenfragmente der Knochenstruktur ermöglicht. Durch die elastische Ausbildung des wenigstens einen Mittelstücks können die Haltestücke in unterschiedlichen Positionen zueinander an der Knochenstruktur angeordnet und gegebenenfalls festgelegt werden, während das oder die Mittelstücke flexibel an die Knochenstruktur angeformt wird. Das erfindungsgemäße Implantatsystem eignet sich in besonderem Maße zur Versorgung geschädigter Knochenstrukturen im Thoraxbereich, also von Rippenknochen, ist aber nicht darauf beschränkt.

Bevorzugte Weiterbildungen des erfindungsgemäßen Implantatsystem finden sich in den entsprechenden Unteransprüchen.

Zuvor wurde bereits eine Ausgestaltung mit einem Mittelstück und zwei an dessen Enden befindlichen Haltestücken beschrieben, es sind aber auch andere Konfigurationen denkbar, so können sich nach den Haltestücken jeweils weitere elastische Mittelstücke anschließen, auf die erneut Haltstücke folgen können. Auch das Weglassen des ein oder anderen jeweiligen Stücks ist denkbar, so dass eine Aneinanderreihung von Mittel- und Haltestücken denkbar ist, die unterschiedlichste Knochenstrukturen mit sich änderndem Umfang und großer Längserstreckung ein- oder auch mehrfach umgreifen oder umschlingen kann.

Bei einer vorteilhaften Weiterbildung des erfindungsgemäßen Implantatsystems, die eine hohe Flexibilität aufweist und eine gute Handhabbarkeit hinsichtlich der Anordnung des Implantatkörpers an der Knochenstruktur zeigt, kann der Implantatkörper mit seiner stabartigen Längserstreckung wenigstens einen Bereich aufweisen, dessen Seitenränder an wenigstens zwei einander gegenüberliegenden Seiten wellenförmig, insbesondere gleichmäßig wellenförmig ausgebildet sind. Dabei können sowohl das wenigstens eine Mittelstück, als auch ein oder mehrere Haltestücke jeweils für sich, aber auch gemeinsam mit der erwähnten Wellenform ausgebildet sein.

Besonders bevorzugt können dabei in einer vereinfacht herstellbaren Weiterbildung das wenigstens eine Mittelstück und die wenigstens zwei Haltestücke im Wesentlichen die gleiche Außenkontur aufweisen, also beispielsweise jeweils die identische gleichmäßige Wellenform an ihren Seitenrändern aufweisen. Die Haltestücke können sich hinsichtlich der Formgebung aber sowohl untereinander, als auch jeweils von dem wenigstens einen Mittelstück unterscheiden.

Eine Möglichkeit die Eigenschaften des Implantatkörpers des erfindungsgemäßen Implantatsystems an die Gegebenheiten der Knochenstruktur und deren Schädigungsgrad anzupassen, besteht bei einer vorteilhaften Weiterbildung darin, dass die Dicke und/oder Länge der Haltstücke und/oder des Mittelstücks verschieden und/oder variierbar ausgebildet sind. Die Variierbarkeit von Halte- bzw. Mittelstücken erlaubt es, dass der betreffende Implantatkörper durch Vermessen der jeweiligen Situation individuell anpassbar ist. Bei vereinfachten Weiterbildungen von Implantatkörpern können beispielsweise die Haltestücke paarweise identisch ausgebildet sein.

Bei einer Weiterbildung, bei der einerseits das Mittelstück ein hohes Maß an Flexibilität mitbringt, und zum anderen die Haltestücke sehr stabil ausgebildet sind, können die Haltestücke mit einer erhöhten Dicke gegenüber dem wenigstens einen Mittelstück ausgebildet sein, so dass sie eine verminderte Nachgiebigkeit zeigen.

Um einerseits das Mittelstück und die Haltestücke des Implantatkörpers insbesondere bei sich unterscheidenden Dicken der Stücke in einer Weise verbinden zu können, die keine unnötigen Vorsprünge oder Stufen aufweist und andererseits, falls notwendig oder gewünscht, die Haltestücke gegenüber dem wenigstens einen Mittelstück durch Abwinkeln bereits mit einer Vorzugsorientierung zu versehen, kann bei einer vorteilhaften Weiterbildung des erfindungsgemäßen Implantatsystems zwischen wenigstens einem der Haltestücke und dem wenigstens einen Mittelstück ein Übergangsbereich vorgesehen sein. Hierzu kann beispielsweise der Übergangsbereich wenigstens einen gegenüber der Längserstreckung gewinkelten Abschnitt aufweisen, der Übergangsbereich kann aber auch eine einfache Verjüngung der Dicke des Querschnitts von der Dicke des wenigstens einen Mittelstücks auf diejenige des jeweiligen Haltestücks bilden. Ebenso ist ein Übergangsbereich denkbar, bei der eine Flachseite des wenigstens einen Mittelstücks und der Haltestücke, insbesondere die Flachseite zur Anlage an der Knochenstruktur, in einer Ebene liegen.

Hinsichtlich ihrer Abmessungen sollen sowohl das Mittelstück, als auch die Haltestücke prinzipiell zunächst keinen Einschränkungen als denjenigen der Umgebung, in der sie eingesetzt werden, unterliegen. Jedoch sind bezüglich einzelner Erstreckungsrichtungen gewisse Abmessungen besonders bevorzugt. So kann die Länge des Implantatkörpers bevorzugt zwischen 1 mm und 300 mm betragen, die Länge des wenigstens einen Mittelstücks kann zwischen 1 mm und 50 mm betragen, dessen Dicke kann zwischen 0,1 mm und 3,5 mm betragen, während die Dicke der Haltestücke 0,5 mm bis 4,5 mm betragen kann. Die bei einer wellenförmigen Ausbildung der Seitenränder des Implantatkörpers gebildeten Ecken können Abrundungen im Bereich zwischen 0,01 und 4,5 mm aufweisen. Überdies ist es denkbar, dass die erwähnten Abmessungen über ihre jeweilige Erstreckungsrichtungen variieren können.

Um den Implantatkörper bei einer zweckmäßigen Weiterbildung des erfindungsgemäßen Implantatsystems in einfacher Weise ortsfest zu halten, kann zwischen dem Implantatkörper und der Knochenstruktur wenigstens ein Befestigungsmittel angeordnet sein.

Bevorzugt ist das wenigstens eine Befestigungsmittel dabei mit jeweils einem oder einer Anzahl von Dornen, Haken oder dergleichen Vorsprüngen gebildet, die eingerichtet sind, in die Knochenstruktur einzudringen. Derartige Vorsprünge können sowohl an dem wenigstens einen Mittelstück, als auch an den beiden Haltestücken vorgesehen sein, und sowohl auf der Knochenstruktur zugewandten Seite, an der sie in die Knochenstruktur eindringen, als auch auf der der Knochenstruktur abgewandten Seite, auf der sie in der Lage sind das umgebende Gewebe zu fixieren, angeordnet sein. Eine andere bevorzugte Weiterbildung kann darin bestehen, dass Schrauben durch hierfür vorgesehene Aufnahmen an einem oder mehreren der Haltestücke greifen und diese derart an der Knochenstruktur festlegen. Auch an Mittelstücken können Aufnahmen zum Durchgriff von Schrauben oder dergleichen Befestigungsmitteln vorgesehen werden,

Um den jeweiligen, gegebenenfalls individuell gefertigten Implantatkörper beispielsweise hinsichtlich spezifisch ausgewählter Materialeigenschaften wie Ermüdungsbeständigkeit, Flexibilität, idealem Elastizitätsmodul oder Festigkeit in geeigneter Weise an die jeweilige Situation anpassen zu können, ist erfindungsgemäß der Implantatkörper des Implantatsystems aus wenigstens einem Hochleistungspolymer oder Kompositen davon ausgebildet. Besonders bevorzugt können dabei das wenigstens eine Mittelstück und die Haltestücke jeweils aus einem thermoplastischen Kunststoff wie einem Polyetheretherketon (PEEK)-Material, einem Polyetherketonketon (PEKK)-Material, einem Polyphenylsulfon (PPSU)-Material, einem Polyethylen (PE)-Material sowie aus sogenannten Compounds aus den oben erwähnten Materialien mit Hydroxylapatit, Tricalciumphosphat, Strontium, Magnesium, und dergleichen ausgebildet sein. Es sind aber auch weitere Materialien denkbar und die vorstehende Aufzählung ist nicht erschöpfend.

Insbesondere bei individueller Fertigung des Implantatkörpers, etwa zur Versorgung komplizierterer Frakturen oder solcher mit schlechterer Heilprognose können das wenigstens eine Mittelstück und die Haltestücke an ihrer jeweils der Knochenstruktur zugewandten Fläche eine dem jeweils zugeordneten Knochenbereich angepasste Oberfläche aufweisen, die eine noch genauere Anpassung gestattet.

Die obige Aufgabe wird auch gelöst durch ein Verfahren zur Herstellung eines Implantatsystem zur Versorgung von Frakturen einer gekrümmten Knochenstruktur nach Anspruch 12.

Ebenso ist es denkbar, die endgültige Form des Implantatkörpers wie auch die endgültige Gestaltung des elastischen wenigstens einen Mittelstücks durch Nachbearbeitung eines in einem Urformverfahren erhaltenen Rohlings des Implantatkörpers herzustellen. Eine solche Nachbearbeitung kann beispielsweise in einem Trennvorgang bestehen, bei dem von dem Rohling mit Hilfe der Schneiden eines Werkzeugs Werkstoffschichten von Spänen zur Änderung der Werkstückform und (oder) Werkstückoberfläche mechanisch abgetrennt werden. Das Spanen kann dabei etwa mittels geometrisch bestimmter Schneiden als Fräsvorgang stattfinden, wobei durch ein derartiges Fertigungsverfahren eine hohe Fertigungsgenauigkeit erreicht werden kann.

Auch in dieser Art und Weise kann ein Implantatsystem zur Verfügung gestellt werden, welches eine Fraktur an einer Knochenstruktur sicher und versagensfrei versorgt und gleichzeitig bei guter Knochenheilung und Schmerzausschaltung eine kontrollierte Bewegung im Frakturspalt ermöglicht.

Die Erfindung wird nachstehend anhand von Ausführungsbeispielen in den Figuren der Zeichnung näher erläutert. In teilweise schematisierter Darstellung zeigen hierbei:
- Fig. 1a: eine perspektivische Seitenansicht einer ersten Ausführungsform des erfindungsgemäßen Implantatsystems mit einem Implantatkörper in gestreckter Form;
- Fig, 1b: eine perspektivische Seitenansicht des Implantatkörpers aus der Fig. 1a in um eine Biegeachse gebogener Form,
- Fig, 1c: eine perspektivische Seitenansicht des an der Knochenstruktur eines Rippenbogens angeordneten Implantatkörper aus der Fig. 1a;
- Fig. 1d: eine perspektivische Seitenansicht des Implantatkörper aus der Fig. 1c in Ansicht von unten;
- Fig. 2a: eine perspektivische Seitenansicht einer zweiten Ausführungsform des erfindungsgemäßen Implantatsystems mit einem Implantatkörper in gestreckter Form, bei welchem an der der Knochenstruktur abgewandten Flachseite die Haltestücke mit Spitzen versehen sind, das Mittelstück aber keine Spitzen aufweist;
- Fig. 2b: eine perspektivische Seitenansicht eines Detailbereichs des einen Haltestücks des Implantatkörpers aus der Fig. 2a;
- Fig. 2c: eine perspektivische Seitenansicht des Implantatkörpers aus den Fig. 2a und 2b bei dem an der der Knochenstruktur abgewandten Flachseite des Implantatkörpers sowohl die Haltestücke als auch das Mittelstück mit Spitzen versehen sind;
- Fig. 3a: eine perspektivische Seitenansicht einer dritten Ausführungsform des erfindungsgemäßen Implantatsystems mit einem Implantatkörper in gestreckter Form, bei welchem an der der Knochenstruktur abgewandten Flachseite das Mittelstück mit Spitzen versehen ist;
- Fig. 3b: eine perspektivische Seitenansicht eines Detailbereichs des Mittelstücks des Implantatkörpers aus der Fig. 3a;
- Fig. 3c: eine perspektivische Seitenansicht des Implantatkörpers aus den Fig. 3a und 3b bei dem an der der Knochenstruktur zugewandten Flachseite des Implantatkörpers nur das Mittelstück mit Spitzen versehen ist und die Flächen des Mittelstücks und der Haltestücke in einer Ebene liegen;
- Fig. 3d: eine perspektivische Seitenansicht eines Detailbereichs des Mittelstücks des Implantatkörpers aus den Fig. 3a-3c;
- Fig. 4: eine perspektivische Seitenansicht einer vierten Ausführungsform des erfindungsgemäßen Implantatsystems mit einem Implantatkörper in gestreckter Form, bei welchem die Haltestücke an ihren Seitenrändern mit einer Wellenform ausgebildet sind, während das Mittelstück sich mit parallelen geraden Seitenrändern erstreckt;
und die
- Fig. 5: eine perspektivische Seitenansicht einer fünften Ausführungsform des erfindungsgemäßen Implantatsystems mit einem Implantatkörper in gestreckter Form, bei welchem die Haltestücke an ihren Seitenrändern im Wesentlichen mit geraden, parallelen Seitenrändern ausgebildet sind, während das Mittelstück sich gerade, aber mit wellenförmigen Seitenrändern erstreckt.

In sämtlichen Figuren sind gleiche bzw. funktionsgleiche Elemente und Vorrichtungen - sofern nichts anderes angegeben ist - mit denselben Bezugszeichen versehen worden.

In den Zeichnungsfiguren 1 bis 5 ist ein Implantatsystem 100 zur Versorgung von Frakturen einer gekrümmten Knochenstruktur 50 gezeigt, das Folgendes umfasst: einen Implantatkörper 10, der vorgesehen ist, an der Knochenstruktur 50 angeordnet zu sein und diese zumindest bereichsweise zu kontaktieren, wobei der Implantatkörper 10 eine stabartige Längserstreckung aufweist, wobei der Implantatkörper 10 wenigstens ein Mittelstück 12 aufweist, welches an beiden Enden zumindest ein jeweiliges Haltestück 14, 16 aufweist, wobei die Haltstücke 14, 16 jeweils derart biege- und/oder verwindungssteif ausgebildet sind, dass eine im Wesentlichen flächige Anlage des jeweiligen Haltestücks 14, 16 entlang eines diesem zugeordneten Bereichs der Knochenstruktur 50 ermöglicht ist, und wobei das wenigstens eine Mittelstück 12 derart elastisch ausgebildet ist, dass die wenigstens zwei Haltestücke 14, 16 einander gegenüber um wenigstens eine Biege- oder Torsionsachse verbiegbar sind.

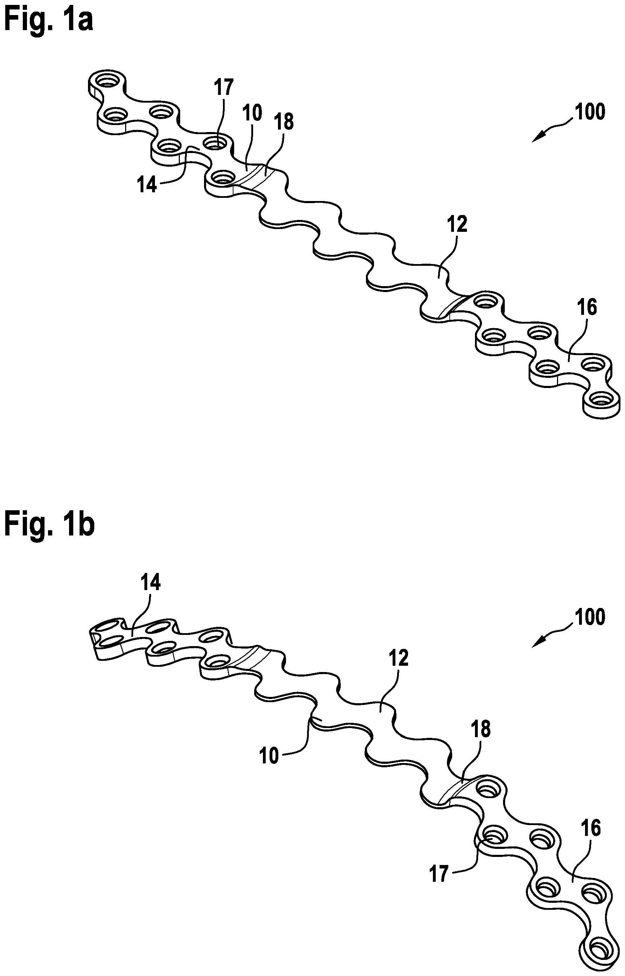

Die Fig. 1a zeigt eine perspektivische Seitenansicht einer ersten Ausführungsform des erfindungsgemäßen Implantatsystems 100 mit einem Implantatkörper 10 in gestreckter Form. Dabei ist der Implantatkörper 10 mit einer stabartigen Längserstreckung ausgebildet und ist sowohl an dem Mittelstück 12, als auch an den beiden die gleiche Außenkontur aufweisenden Haltestücken 14, 16 mit Seitenrändern versehen, die an zwei einander gegenüberliegenden Seiten gleichmäßig wellenförmig ausgebildet sind. Durch die Wellenform sind an den Seitenrändern jeweils abwechselnd in einander übergehende vor- und zurückspringende Abschnitte ausgebildet, wobei ein zurückspringender Abschnitt des einen Seitenrandes mit einem vorspringenden Abschnitt des gegenüberliegenden Seitenrandes übereinstimmt. In den vorspringenden Abschnitten der Haltestücke 14, 16 sind senkrecht zur Längserstreckung Aufnahmen 17 zur Anordnung von Befestigungsschrauben (nicht dargestellt) eingebracht. Zwischen den Haltestücken 14, 16 und dem Mittelstück 12 ist jeweils ein Übergangsbereich 18 angeordnet, an denen ein stufenfreier Übergang zwischen den senkrecht zur Längserstreckung dickeren Haltestücken 14, 16 und dem dünneren Mittelstück 12 hergestellt wird. Die Übergangsstücke überführen die Niveaus der Flachseiten der Haltestücke 14, 16, die, wenn der Implantatkörper 10 nicht gebogen ist, miteinander fluchten, in dasjenige des Mittelstücks 12.

Die Fig. 1b zeigt eine perspektivische Seitenansicht des Implantatkörpers 10 aus der Fig. 1a in um eine Biegeachse gebogener Form, wobei die Biegeachse (nicht dargestellt) in der durch das Mittelstück 12 aufgespannten Ebene mittig und quer zur Längserstreckung des Implantatkörpers 10 verläuft.

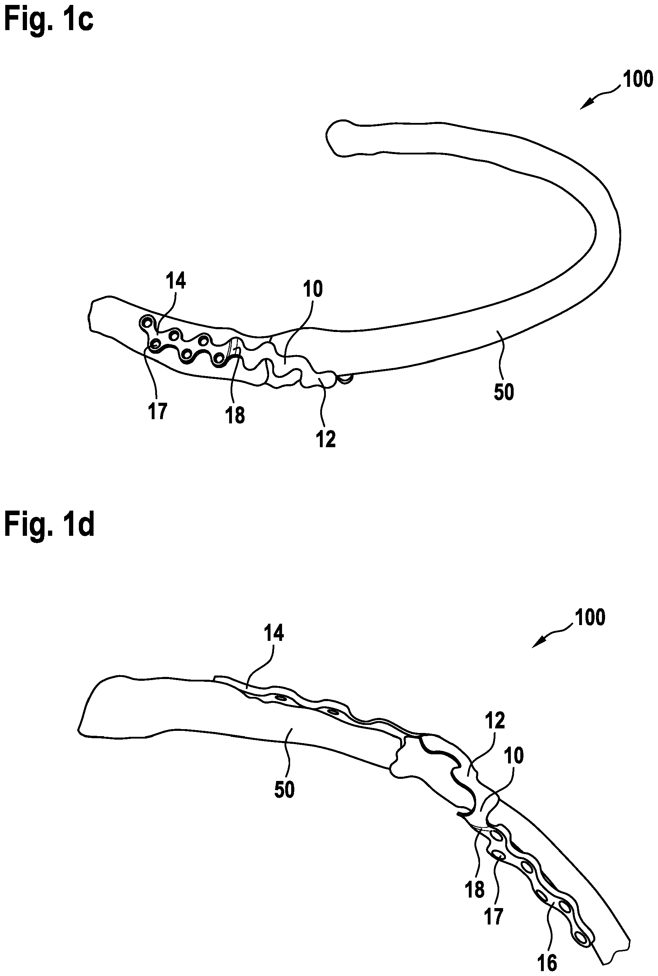

Die Fig. 1c zeigt eine perspektivische Seitenansicht des an der geschädigten Knochenstruktur 50 eines Rippenbogens angeordneten Implantatkörper 10 aus der Fig. 1a. Die Schädigung der Knochenstruktur 50 in Form einer Fraktur ist dabei gut zu erkennen. Weiter erkennt man eines der Haltestücke 14, welches an der Knochenstruktur 50 festgelegt ist und außerdem das um eine entlang der Knochenstruktur verlaufende Torsionsachse derart verdrehte Mittelstück 12, dass der Implantatkörper 10 die Knochenstruktur 50 um etwa 180° umschlingt. Die Fig. 1d zeigt eine perspektivische Seitenansicht des Implantatkörpers aus der Fig. 1c in Ansicht von unten, in der beide Haltestücke 14, 16 und das Mittelstück 12 zu erkennen sind. In den Fig, 1c und 1d erkennt man weiter, dass die Haltestücke 14, 16 von der Fraktur selbst beabstandet sind und das Mittelstück 12 diesen Bereich derart umschlingt oder umgreift, dass unter Begünstigung knöcherner Heilung eine kontrollierte Bewegung und Lastverteilung des dynamischen Brustraums begünstigt wird.

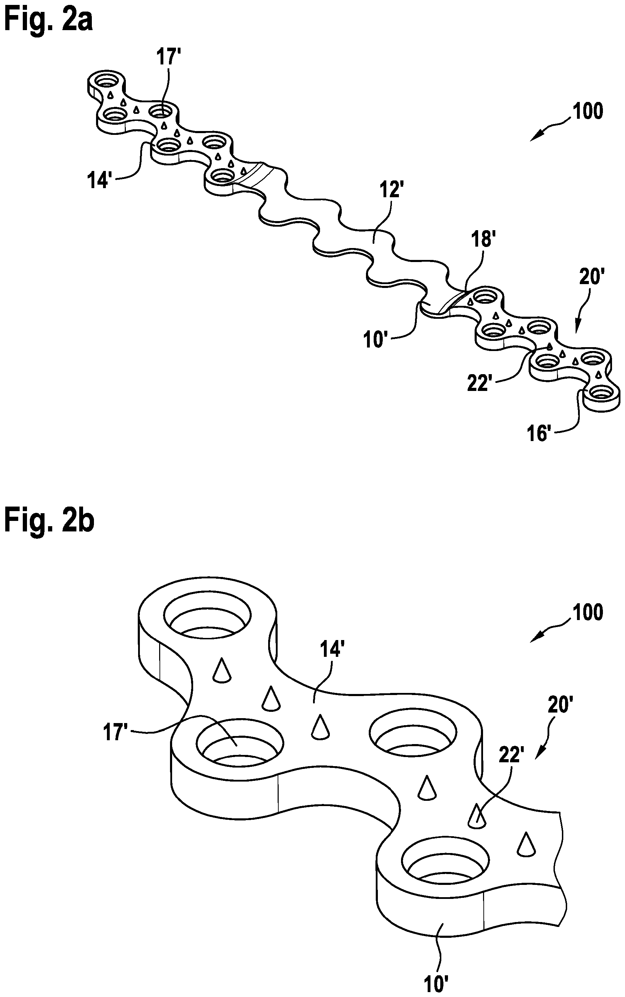

Die Fig. 2a zeigt eine perspektivische Seitenansicht einer zweiten Ausführungsform des erfindungsgemäßen Implantatsystems 100 mit einem Implantatkörper 10' in gestreckter Form, bei welchem an der der Knochenstruktur 50 (nicht dargestellt) abzuwendenden Flachseite die Haltestücke 14', 16' mit Spitzen 22' als Befestigungsmittel 20' versehen sind, das Mittelstück 12 an seiner Flachseite jedoch keine Spitzen 22' aufweist. Das Mittelstück 12' und die Haltestücke 14', 16' sind bei dem Implantatkörper 10', der erneut eine stabartige Längserstreckung aufweist, wiederum mit Seitenrändern versehen, die an zwei einander gegenüberliegenden Seiten gleichmäßig wellenförmig ausgebildet sind. Auch hier ist zwischen den Haltestücken 14', 16' und dem Mittelstück 12' jeweils ein Übergangsbereich 18' angeordnet, an denen ein stufenfreier Übergang zwischen den dickeren Haltestücken 14', 16' und dem dünneren Mittelstück 12' hergestellt wird.

Die Fig. 2b zeigt dabei eine perspektivische Seitenansicht eines Detailbereichs des einen Haltestücks 14' des Implantatkörpers 10' aus der Fig. 2a, in der man erkennt, dass die Spitzen 22' in Erstreckungsrichtung des Implantatkörpers 10' entlang einer Linie zwischen den vor- und zurückspringenden Abschnitten in Gruppen zu je drei Spitzen 22' angeordnet sind. Auch hier sind in den vorspringenden Abschnitten der Haltestücke 14', 16' sind senkrecht zur Längserstreckung Aufnahmen 17' zur Anordnung von Befestigungsschrauben (nicht dargestellt) eingebracht.

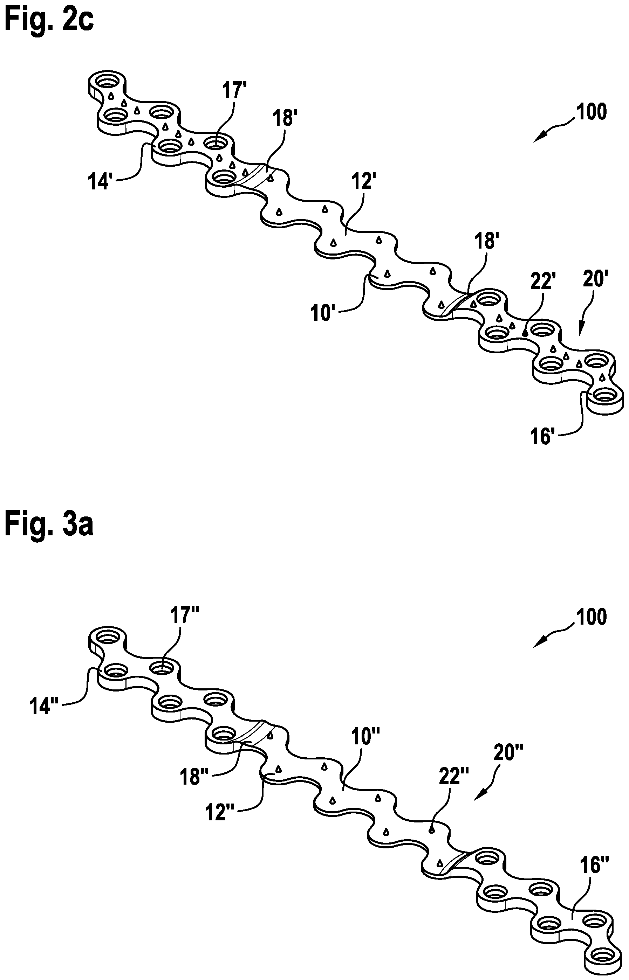

Die Fig. 2c zeigt eine perspektivische Seitenansicht des Implantatkörpers 10' aus den Fig. 2a und 2b bei dem an der der Knochenstruktur abgewandten Flachseite des Implantatkörpers 10' sowohl die Haltestücke 14', 16'als auch das Mittelstück 12' mit Spitzen 22' versehen sind, wodurch das den Implantatkörper 10' umgebende Gewebe ortsfest gehalten wird.

Die Fig. 3a zeigt eine perspektivische Seitenansicht einer dritten Ausführungsform des erfindungsgemäßen Implantatsystems 100 mit einem Implantatkörper 10" in gestreckter Form, bei welchem an der der Knochenstruktur abgewandten Flachseite das Mittelstück 12" mit Spitzen 22" versehen ist. Erneut ist dabei der Implantatkörper 10" mit einer stabartigen Längserstreckung ausgebildet und ist sowohl an dem Mittelstück 12", als auch an den beiden die gleiche Außenkontur aufweisenden Haltestücken 14", 16" mit Seitenrändern versehen, die an zwei einander gegenüberliegenden Seiten gleichmäßig wellenförmig ausgebildet sind. Durch die Wellenform sind an den Seitenrändern jeweils abwechselnd in einander übergehende vor- und zurückspringende Abschnitte ausgebildet, wobei ein zurückspringender Abschnitt des einen Seitenrandes mit einem vorspringenden Abschnitt des gegenüberliegenden Seitenrandes übereinstimmt. In den vorspringenden Abschnitten der Haltestücke 14", 16" sind senkrecht zur Längserstreckung Aufnahmen 17" zur Anordnung von Befestigungsschrauben (nicht dargestellt) eingebracht.

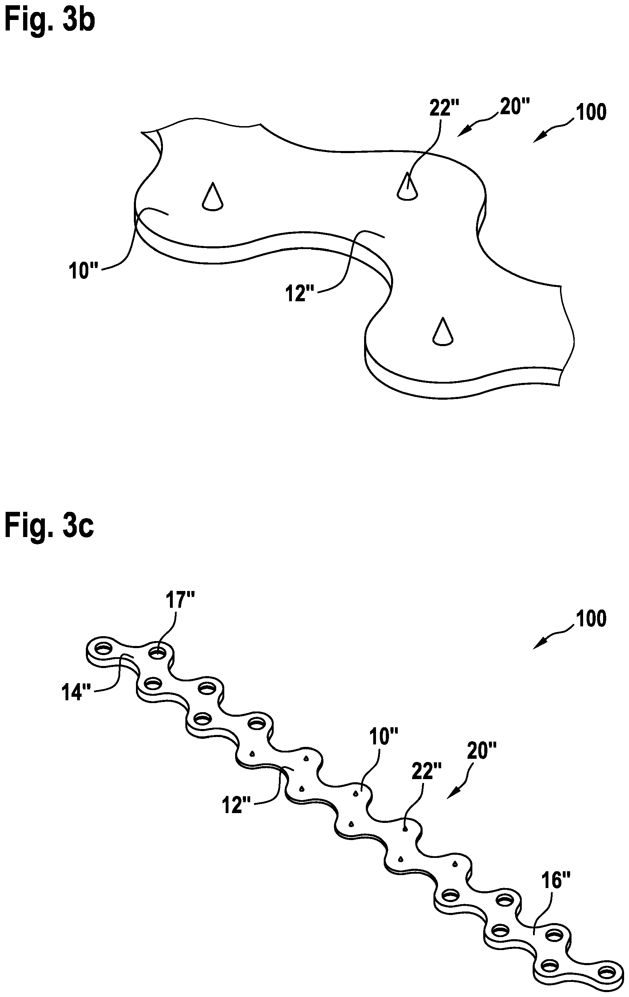

Die Fig. 3b zeigt eine perspektivische Seitenansicht eines Detailbereichs des Mittelstücks 12" des Implantatkörpers 10" aus der Fig. 3a. Dabei erkennt man, dass die Spitzen 22" an dem Mittelstück 12" jeweils einzeln an jedem vorspringenden Abschnitt angeordnet sind.

Die Fig. 3c zeigt eine perspektivische Seitenansicht des Implantatkörpers 10" aus den Fig. 3a und 3b bei dem auch an der der Knochenstruktur zugewandten Flachseite des Implantatkörpers 10 " nur das Mittelstück 12" mit Spitzen 22" versehen ist, wobei die Flächen des Mittelstücks 12" und der Haltestücke 14", 16" in einer Ebene liegen.

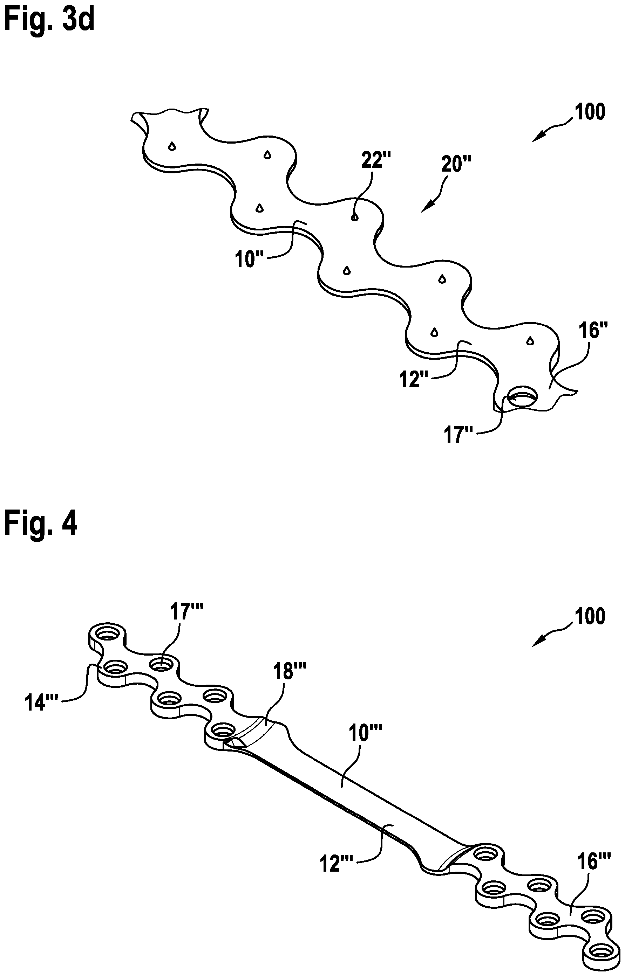

Die Fig. 3d zeigt eine perspektivische Seitenansicht eines Detailbereichs des Mittelstücks 12" des Implantatkörpers 10" aus den Fig. 3a-3c.

Die Fig. 4 zeigt eine perspektivische Seitenansicht einer vierten Ausführungsform des erfindungsgemäßen Implantatsystems 100 mit einem Implantatkörper 10‴ in gestreckter Form, bei welchem die Haltestücke 14‴, 16‴ an ihren Seitenrändern mit einer Wellenform ausgebildet sind, während das Mittelstück 12‴ sich mit parallelen geraden Seitenrändern erstreckt. Auch hierbei ist zwischen den Haltestücken 14"', 16‴ und dem Mittelstück 12‴ jeweils ein Übergangsbereich 18‴ angeordnet. Hinsichtlich der Wellenform der Haltestücke 14"', 16‴ und der Aufnahmen 17‴ für Befestigungsmittel kann auf das zu den Fig. 1 bis 3 Gesagte verwiesen werden.

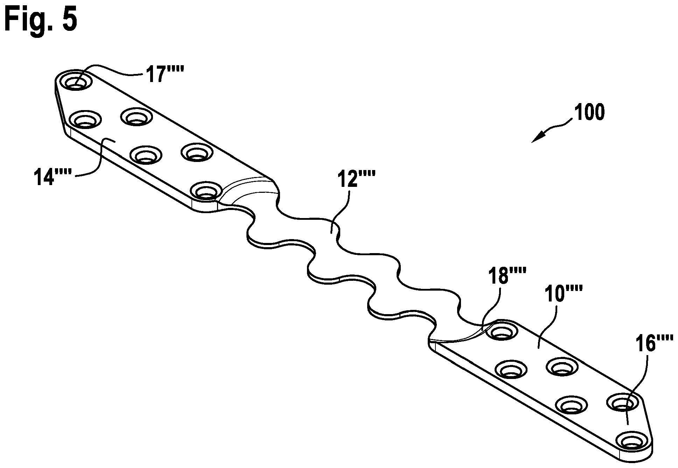

Die Fig. 5 zeigt eine perspektivische Seitenansicht einer fünften Ausführungsform des erfindungsgemäßen Implantatsystems 100 mit einem Implantatkörper 10"" in gestreckter Form, bei welchem die Haltestücke 14"", 16"" an ihren Seitenrändern im Wesentlichen geraden, parallelen Seitenrändern ausgebildet sind, während das Mittelstück 12"" sich gerade, aber mit wellenförmigen Seitenrändern erstreckt. Die Aufnahmen 17"" zur Anordnung von Befestigungsschrauben weisen dabei dasselbe Muster wie bei einer wellenförmigen Ausbildung des Seitenrandes auf und an den Enden der Haltestücke 14"", 16"" laufen deren Seitenränder jeweils an einer Aufnahme 17"" zusammen.

Das erfindungsgemäße Implantatsystem 100 löst dabei das eingangs genannte Problem durch die Kombination eines innovativen und flexiblen Designs und der spezifisch ausgewählten Materialeigenschaften wie Ermüdungsbeständigkeit, Flexibilität, idealen Elastizitätsmoduls, Materialfestigkeit. Somit kann eine kontrollierte Lastverteilung als auch kontrollierte Bewegung des dynamischen Brustraums sichergestellt werden, welches die knöcherne Heilung im Gegensatz zu einem steiferen metallischen Systems begünstigt.

## Patentansprüche

1. Implantatsystem (100) aus wenigstens einem Hochleistungspolymer oder Kompositen davon zur Versorgung von Frakturen einer gekrümmten Knochenstruktur (50), umfassend:
einen Implantatkörper (10, 10', 10", 10"', 10""), der vorgesehen ist, an der Knochenstruktur (50) angeordnet zu sein und diese zumindest bereichsweise zu kontaktieren, wobei der Implantatkörper (10, 10', 10", 10"', 10"") eine stabartige Längserstreckung aufweist,
wobei der Implantatkörper (10, 10', 10", 10"', 10"") ein Mittelstück (12, 12', 12", 12‴, 12"") aufweist, welches an beiden Enden ein jeweiliges Haltestück (14, 14', 14", 14‴, 14ʺʺ, 16, 16', 16", 16‴, 16"") aufweist,
wobei die Haltestücke (14, 14', 14", 14"', 14"", 16, 16', 16", 16"', 16"") jeweils derart biege- und/oder verwindungssteif ausgebildet sind, dass eine im Wesentlichen flächige Anlage des jeweiligen Haltestücks (14, 14', 14", 14"', 14"", 16, 16', 16", 16‴, 16"") entlang eines diesem zugeordneten Bereichs der Knochenstruktur (50) ermöglicht ist, und
wobei das Mittelstück (12, 12', 12", 12"', 12"") derart elastisch ausgebildet ist, dass die wenigstens zwei Haltestücke (14, 14', 14", 14‴, 14"", 16, 16', 16", 16‴, 16"") einander gegenüber um eine Biegeachse und eine Torsionsachse verbiegbar sind, so dass die Knochenstruktur (50) von dem Mittelstück (12, 12', 12", 12"', 12"") zumindest bereichsweise umschlingbar ist.

2. Implantatsystem (100) nach Anspruch 1, wobei der Implantatkörper (10) mit seiner stabartigen Längserstreckung wenigstens einen Bereich aufweist, dessen Seitenränder an wenigstens zwei einander gegenüberliegenden Seiten wellenförmig, insbesondere gleichmäßig wellenförmig, ausgebildet sind.

3. Implantatsystem (100) nach einem der Ansprüche 1 oder 2, wobei das Mittelstück (12, 12', 12", 12‴, 12"") und die zwei Haltestücke (14, 16) im Wesentlichen die gleiche Außenkontur aufweisen.

4. Implantatsystem (100) nach einem der vorhergehenden Ansprüche, wobei die Dicke und/oder Länge der Haltestücke (14,16) und/oder des Mittelstücks (12, 12', 12", 12"', 12"") sich unterscheiden.

5. Implantatsystem (100) nach einem der vorhergehenden Ansprüche, wobei die Haltestücke (14, 16) mit einer erhöhten Dicke gegenüber dem Mittelstück (12, 12', 12", 12‴, 12"") ausgebildet sind.

6. Implantatsystem (100) nach einem der vorhergehenden Ansprüche, wobei zwischen wenigstens einem der Haltestücke (14, 16) und dem Mittelstück (12, 12', 12", 12‴, 12"") ein Übergangsbereich (18, 18', 18", 18"', 18"") vorgesehen ist.

7. Implantatsystem (100) nach Anspruch 6, wobei der Übergangsbereich (18, 18', 18", 18‴, 18"") wenigstens einen gegenüber der Längserstreckung gewinkelten Abschnitt aufweist.

8. Implantatsystem (100) nach einem der vorhergehenden Ansprüche, wobei an dem Implantatkörper (10', 10") wenigstens ein Befestigungsmittel (20', 20") angeordnet ist.

9. Implantatsystem (100) nach Anspruch 8, wobei das Befestigungsmittel (20', 20") mit einem oder einer Anzahl von Dornen, Spitzen (22', 22"), Haken oder dergleichen Vorsprüngen gebildet ist, die eingerichtet sind, in eine knöcherne Struktur der Knochenstruktur (50) einzudringen oder den Implantatkörper (10', 10") umgebendes Gewebe ortsfest zu halten.

10. Implantatsystem (100) nach einem der vorhergehenden Ansprüche, wobei das wenigstens eine Hochleistungspolymer aus einem thermoplastischen Kunststoff wie einem Polyetheretherketon (PEEK)-Material, einem Polyetherketonketon (PEKK)-Material , einem Polyphenylsulfon (PPSU)-Material, einem Polyethylen (PE)-Material sowie Compounds aus den erwähnten Materialien mit Hydroxylapatit, Tricalciumphosphat, Strontium, Magnesium, und dergleichen ausgebildet ist.

11. Implantatsystem (100) nach einem der vorhergehenden Ansprüche, wobei das Mittelstück (12, 12', 12", 12"', 12ʺʺ) und die Haltestücke (14, 14', 14", 14"', 14ʺʺ, 16, 16', 16", 16"', 16"") an ihrer jeweils der Knochenstruktur (50) zugewandten Fläche eine dem jeweils zugeordneten Knochenbereich angepasste Oberfläche aufweisen.

12. Verfahren zur Herstellung eines Implantatsystem (100) nach einem der vorgenannten Ansprüche zur Versorgung von Frakturen einer gekrümmten Knochenstruktur (50), umfassend zumindest die folgenden Herstellungsschritte:
- Bereitstellen einer Implantatkörperform, die in wenigstens drei verschiedene Bereiche unterteilt ist, einen Mittelbereich und jeweils einen an dessen Enden befindlichen Endbereich;
- Einspritzen jeweils wenigstens eines Hochleistungspolymers in jeden der Bereiche der Implantatkörperform;
- Erkalten und/oder Vernetzen der Hochleistungspolymere in den verschiedenen Bereichen unter Verbinden der Bereiche zu demstabförmigen, biegsamen Implantatkörper (10, 10', 10", 10‴, 10ʺʺ) mit dem elastischen Mittelstück (12, 12', 12'', 12‴, 12"") und den zwei Haltestücken (14, 14', 14", 14‴, 14"", 16, 16', 16", 16"', 16""), die gegenüber einander um die eine Biegeachse und die eine Torsionsachse verbiegbar sind;
- Entformen des Implantatkörpers (10) aus der Implantatkörperform.

## Claims

1. Implant system (100) consisting of at least one high performance polymer or composites thereof for treating fractures of a curved bone structure (50), comprising an implant body (10, 10', 10'', 10‴, 10""), which is provided to be arranged on the bone structure (50) and to make contact with it at least in areas, wherein the implant body (10, 10', 10'', 10''', 10"") has a rod-like longitudinal extension,
wherein the implant body (10, 10', 10'', 10‴, 10"") has a centre piece (12, 12', 12'', 12''', 12"") which has a respective retaining piece (14, 14', 14'', 14‴, 14"", 16, 16', 16'', 16‴, 16"") on both ends,
wherein the retaining pieces (14, 14', 14'', 14''', 14"", 16, 16', 16'', 16‴, 16"") are respectively designed to be resistant to bending and/or torsion in such a way that substantially planar contact of the respective retaining piece (14, 14', 14'', 14‴, 14"", 16, 16', 16'', 16‴, 16"") along a region of the bone structure (50) associated therewith is enabled, and
wherein the centre piece (12, 12', 12'', 12‴, 12"") is designed to be elastic in such a way that the at least two retaining pieces (14, 14', 14'', 14‴, 14"", 16, 16', 16'', 16‴, 16"") can be bent relative to one another about a bending axis and a torsion axis such that the bone structure (50) can be embraced by the centre piece (12, 12', 12", 12‴, 12"") at least in areas.

2. Implant system (100) according to claim 1, wherein the implant body (10) with its rod-like longitudinal extension has at least one region, the side edges of which are formed in an undulating manner, in particular a uniformly undulating manner, on at least two opposites sides.

3. Implant system (100) according to one of Claims 1 or 2, wherein the centre piece (12, 12', 12'', 12‴, 12"") and the two retaining pieces (14, 16) have substantially the same outer contour.

4. Implant system (100) according to one of the preceding claims, wherein the thickness and/or length of the retaining pieces (14, 16) and/or centre piece (12, 12', 12'', 12‴, 12"") vary.

5. Implant system (100) according to one of the preceding claims, wherein the retaining pieces (14, 16) are designed with an increased thickness compared to the centre piece (12, 12', 12", 12‴, 12"").

6. Implant system (100) according to one of the preceding claims, wherein a transition region (18, 18', 18'', 18''', 18"") is provided between at least one of the holding pieces (14, 16) and the centre piece (12, 12', 12'', 12‴, 12"").

7. Implant system (100) according to claim 6, wherein the transition region (18, 18', 18'', 18‴, 18"") has at least one section angled relative to the longitudinal extension.

8. Implant system (100) according to one of the preceding claims, wherein at least one fastening means (20', 20'') is arranged on the implant body (10', 10'').

9. Implant system (100) according to claim 8, wherein the fastening means (20', 20'') is formed with one or a number of spikes, points (22', 22''), hooks or such protrusions, which are designed to penetrate into a bony structure of the bone structure (50) or hold tissue surrounding the implant body (10', 10'') in place.

10. Implant system (100) according to one of the preceding claims, wherein the at least one high performance polymer is made of a thermoplastic material such as a polyetheretherketone (PEEK) material, polyetherketoneketone (PEKK) material, a polyphenylsulfone (PPSU) material, a polyethylene (PE) material as well as compounds of the aforementioned materials with hydroxyapatite, tricalcium phosphate, strontium, magnesium, and the like.

11. Implant system (100) according to one of the preceding claims, wherein the centre piece (12, 12', 12'', 12‴, 12"") and the retaining pieces (14, 14', 14'', 14''', 14"", 16, 16', 16", 16‴, 16"") have a surface adapted to the respectively associated bone region on their surface respectively facing the bone structure (50).

12. Method for producing an implant system (100) according to one of the preceding claims for treating fractures of a curved bone structure (50), comprising at least the following production steps:
- providing an implant body mould which is divided into at least three different regions, a central region and an end region located at each of its ends;
- injecting at least one high performance polymer into each of the regions of the implant body mould;
- cooling and/or cross-linking the high performance polymers in the various regions while joining the regions to form the rod-shaped, flexible implant body (10, 10', 10'', 10‴, 10"") with the elastic centre piece (12, 12', 12", 12‴, 12"") and the two retaining pieces (14, 14', 14'', 14‴, 14"", 16, 16', 16'', 16‴, 16""), which can be bent relative to one another about the one bending axis and the one torsion axis;
- removing the implant body (10) from the implant body mould.

## Revendications

1. Système d'implant (100) constitué d'au moins un polymère à haute performance ou de composites de celui-ci, pour le traitement de fractures d'une structure d'os incurvée (50), comprenant :
un corps d'implant (10, 10', 10'', 10‴, 10"") qui est destiné à être agencé sur la structure d'os (50) et à venir en contact avec celle-ci au moins par sections, dans lequel le corps d'implant (10, 10', 10'', 10''', 10'''') comporte une extension longitudinale en forme de tige,
dans lequel le corps d'implant (10, 10', 10'', 10‴, 10"") comporte une pièce centrale (12, 12', 12'', 12‴, 12"") qui comporte une pièce de maintien (14, 14', 14'', 14‴, 14"", 16, 16', 16'', 16''', 16'''') respective aux deux extrémités,
dans lequel les pièces de maintien (14, 14', 14", 14‴, 14"", 16, 16', 16", 16‴, 16"") sont respectivement conçues de manière à résister à la flexion et/ou à la torsion de telle sorte qu'une application sensiblement à plat de la pièce de maintien (14, 14', 14", 14‴, 14"", 16, 16', 16'', 16''', 16'''') respective est permise le long d'une région de la structure d'os (50) associée à celle-ci, et
dans lequel la pièce centrale (12, 12', 12", 12‴, 12"") est conçue de manière élastique de telle sorte que les au moins deux pièces de maintien (14, 14', 14'', 14‴, 14"", 16, 16', 16", 16‴, 16"") sont aptes à être être pliées l'une par rapport à l'autre autour d'un axe de flexion et d'un axe de torsion, de sorte que la structure d'os (50) est apte à s'enrouler autour de la pièce centrale (12, 12', 12'', 12''', 12"") au moins par sections.

2. Système d'implant (100) selon la revendication 1, dans lequel le corps d'implant (10) avec son extension longitudinale en forme de tige comporte au moins une région dont les bords latéraux sont configurés de manière ondulée, en particulier de manière uniformément ondulée, sur au moins deux côtés mutuellement opposés.

3. Système d'implant (100) selon l'une des revendications 1 ou 2, dans lequel la pièce centrale (12, 12', 12'', 12‴, 12"") et les deux pièces de maintien (14, 16) ont sensiblement le même contour extérieur.

4. Système d'implant (100) selon l'une des revendications précédentes, dans lequel l'épaisseur et/ou la longueur des pièces de maintien (14, 16) et/ou de la pièce centrale (12, 12', 12'', 12''', 12'''') sont différentes.

5. Système d'implant (100) selon l'une des revendications précédentes, dans lequel les pièces de maintien (14, 16) sont réalisées en ayant une épaisseur plus grande que la pièce centrale (12, 12', 12'', 12‴, 12"").

6. Système d'implant (100) selon l'une des revendications précédentes, dans lequel une région de transition (18, 18', 18'', 18‴, 18"") est prévue entre au moins une des pièces de maintien (14, 16) et la pièce centrale (12, 12', 12'', 12‴, 12"").

7. Système d'implant (100) selon la revendication 6, dans lequel la région de transition (18, 18', 18'', 18‴, 18"") comporte au moins une partie formant un angle par rapport à l'extension longitudinale.

8. Système d'implant (100) selon l'une des revendications précédentes, dans lequel au moins un moyen de fixation (20', 20'') est agencé sur le corps d'implant (10', 10'').

9. Système d'implant (100) selon la revendication 8, dans lequel le moyen de fixation (20', 20") est formé avec un ou plusieurs éléments parmi des ardillons, des pointes (22', 22''), des crochets ou des saillies analogues, qui sont configurés pour pénétrer dans une structure osseuse de la structure d'os (50) ou pour maintenir en position du tissu entourant le corps d'implant (10', 10'').

10. Système d'implant (100) selon l'une des revendications précédentes, dans lequel le au moins un polymère à haute performance est constitué d'une matière thermoplastique telle qu'une polyétheréthercétone (PEEK), une polyéthercétonecétone (PEKK), un polyphénylènesulfone (PPSU), un polyéthylène (PE) ainsi que des compositions constituées des matières précitées avec de l'hydroxyapatite, du phosphate tricalcique, du strontium, du magnésium et analogue.

11. Système d'implant (100) selon l'une des revendications précédentes, dans lequel la pièce centrale (12, 12', 12'', 12''', 12'''') et les pièces de maintien (14, 14', 14'', 14‴, 14"", 16, 16', 16'', 16''', 16'''') comportent, au niveau de leur face respectivement dirigée vers la structure d'os (50), une surface adaptée à la région osseuse respectivement associée.

12. Procédé de fabrication d'un système d'implant (100) selon l'une des revendications précédentes pour le traitement de fractures d'une structure d'os incurvée (50), comprenant au moins les étapes de fabrication suivantes consistant à :
- fournir un moule de corps d'implant qui est divisé en au moins trois zones différentes, une zone centrale et une zone d'extrémité située à chacune de ses extrémités ;
- injecter respectivement au moins un polymère à haute performance dans chacune des zones du moule de corps d'implant ;
- refroidir et/ou réticuler les polymères à haute performance dans les différentes zones en assemblant les zones au corps d'implant flexible en forme de tige (10, 10', 10'', 10‴, 10"") avec la pièce centrale élastique (12, 12', 12", 12‴, 12"") et les deux pièces de retenue (14, 14', 14'', 14''', 14"", 16, 16', 16'', 16''', 16"") qui sont aptes à être pliées l'une par rapport à l'autre autour de l'axe de flexion et de l'axe de torsion ;
- retirer le corps de l'implant (10) du moule de corps d'implant.
